Europäisches Patentamt

European Patent Office

Office européen des brevets

(19)

(11) Publication number: **0 056 761**
**B1**

# EUROPEAN PATENT SPECIFICATION

(12)

(45) Date of publication of patent specification: **29.02.84**

(51) Int. Cl.³: **A 61 K 39/29, C 12 N 5/00**

(21) Application number: **82400074.9**

(22) Date of filing: **15.01.82**

(54) Method of producing hepatitis B surface antigen.

(30) Priority: **16.01.81 US 225555**

(43) Date of publication of application:
**28.07.82 Bulletin 82/30**

(45) Publication of the grant of the patent:
**29.02.84 Bulletin 84/9**

(84) Designated Contracting States:
**AT BE CH DE FR GB IT LI LU NL SE**

(56) References cited:
EP - A - 0 025 745
EP - A - 0 028 567
EP - A - 0 028 987
EP - A - 0 037 330

BIOLOGICAL ABSTRACTS, volume 70, (1980)
ref. 75607 & J. MED. Virol. 5(3): 257-264 1980
J.A. COPELAND et al. "Hepatitis B surface
antigen production as a growth cycle-related
terminal event in PLC/PRF/5 hepatoma cells"

(73) Proprietor: MERCK & CO. INC.
126, East Lincoln Avenue P.O. Box 2000
Rahway New Jersey 07065 (US)

(72) Inventor: Markus, Henri Z.
1517 Thornberry Road
Wyncote Pennsylvania 19095 (US)
Inventor: McAleer, William J.
717 Marietta Drive
Ambler Pennsylvania 19002 (US)

(74) Representative: Corre, Jacques Denis Paul et al,
Cabinet Regimbeau 26, Avenue Kléber
F-75116 Paris (FR)

(56) References cited:
BIOLOGICAL ABSTRACTS, volume 63, (1977)
ref. 52500 & Br. J. Cancer 34(5): 509-515 1976
(recd. 1977) G.M. MACNAB et al. "Hepatitis B
surface antigen produced by a human hepatoma
cell line"

CHEMICAL ABSTRACTS, volume 91, (1979),
page 197, abstract 118970y COLUMBUS OHIO
(US) & Virology 1979, 96(2), 652-5 J.
MONJARDINO et al. "Polypeptide profile of
HB2Ag excreted by a human hepatoma cell line"

₁⑤⑥ References cited:

CHEMICAL ABSTRACTS, volume 92, 1980,
page 442, abstract 92540b COLUMBUS OHIO
(US) & J. Virol. 1979, 32(3), 796-802 P.L.
MARION et al. "Polypeptides of hepatitis B virus
surface antigen produced by a hepatoma cell
line"

CHEMICAL ABSTRACTS, volume 93, 1980,
page 337, abstract 164212s COLUMBUS OHIO
(US) & Proc. Natl. Acad. Sci. U.S.A. 1980, 77(8)
4549-53 M.F. DUBOIS et al. "Excretion of
hepatitis B surface antigen particles from mouse
cells transformed with cloned viral DNA"

NATURE, volume 282, December 6, 1979 D.P.
ADEN et al. "Controlled synthesis of HbsAG in a
differentiated human liver carcinomaderived cell
line" pages 615-616

NATURE, volume 282, December 6, 1979 J.
SKELLY et al. "Hepatitis B surface antigen
produced by a human hepatoma cell line" pages
617-618

### Method of producing hepatitis B surface antigen

**Background of the Invention**

Hepatitis B surface antigen (HBsAg) has been shown to be effective as a vaccine against hepatitis B disease. The usual source of this antigen is plasma obtained from donors, e.g., by phasmaphoresis. As a result the supply of plasma containing this antigen is uncertain and expensive as most plasma is free of HBsAg.

It has been known heretofore to grow *in vitro* on hollow fiber capillary units tissue cultures of cells which shed HBsAg. The hollow fiber units used heretofore have had a molecular weight cut off of 100,000 or greater.

**Objects of the Invention**

It is an object of the present invention to provide an improved *in vitro* tissue culture method for preparing HBsAg. Another object is to provide a method for preparing HBsAg in higher yield and at a faster rate. These and other objects of the present invention will be apparent from the following description.

**Summary of the Invention**

Hepatitis B surface antigen (HBsAg) is produced *in vitro* in high titer and purity from tissue cultures of cells that shed HBsAg. The cells are grown on hollow fiber capillary units having a molecular weight cut-off of about 10,000.

**Detailed Description**

The present invention relates to a method of producing hepatitis B surface antigen (HBsAg) *in vitro* on hollow fiber capillary units having a molecular weight cut-off of about 10,000.

It has now been found that the yield of HBsAg from cells which shed HBsAg is increased markedly and at a faster rate when the cells are grown in cell culture *in vitro* on hollow fiber capillary units having a molecular weight cut-off of about 10,000. Higher yields are obtained when the growth cycle has two different elevated temperatures. Each temperature is above room temperature and the first temperature is higher than the second temperature. The first temperature is from 35 to 38°C while the second temperature is from 30 to 34°C. Preferably the first temperature is about 37°C and the second temperature is about 32°C.

It has been found in addition that higher yields are obtained when caffeine is present in the *in vitro* cell culture nutrient medium in an amount effective to improve yield of HBsAg or when the *in vitro* cell culture is effected on a permeable membrane. The caffeine may be present in a level at which it exerts a detectable improvement in yield up to a level above which it exerts a toxic effect.

Typically the caffeine is employed at from 0.0001M to 0.007M, preferably at from 0.0001M to 0.0003M.

Any cells which shed HBsAg may be used in the process of the present invention. Examples of such cells are some human hepatomas, high yielding clones from such human hepatomas, and liver cells from hepatitis B infected chimpanzees.

The human hepatoma tissue is grown *in vitro* in the presence of a nutrient medium. By a nutrient medium is meant one which permits growth of cells *in vitro*. Some specific nutrient media are, for example, Medium 199, Morgan et al., Proc. Soc. Exp. Biol. & Med.; 73:1—8, 1950; Basal Medium Eagle, Eagle Science, 122, 501—504, 1955; Morton, In Vitro, Vol. 6, No. 2, pp. 89—108. 1970; Dulbecco's Modified Eagle's Medium, Dulbecco et al., Virology, 8, 396, 1959; Smith et al., J. Virol. 12, 185—196, 1960; Morton, op cit. Minimum Essential Medium (Eagle), Science, 130, 432 (1959) and RPMI Media, Moore et al., J.A.M.A. 199, 519—524, 1967; Morton, op. cit.

The hollow fiber capillary unit is formed of a plurality of anisotropic hollow fiber membranes which provide a matrix for the culture of cells and organ explants. A bundle of these capillaries forms a three demensional vascular system which permits controlled perfusion of cell aggregates with nutrients, as well as exchange of excreted substances. The capillaries consist of a sponge-like body with a very thin (0.5 — 1 $\mu$m), smooth layer of extremely fine, controlled pore size (approximate range: 0.001 — 0.01 $\mu$m) on the lumen side. From that surface outward, the pores become increasingly larger 5—10 $\mu$m at the perimeter. This structure provides a unique combination of selectively and extremely high permeability to liquids even at very low or zero pressure. The choice of desired membrane "cut-off" levels offers selective control of macromolecule transport. The open exterior of the capillaries presents a large surface for cell attachment and allows cells to penetrate toward the barrier at the lumen. The anisotropic fiber membranes may be prepared as described in U.S. patent 3,615,024. The bundle of hollow fiber capillary units may be prepared as described in U.S. patent 3,821,087.

While many types of cells have been grown on hollow fiber capillary units, e.g., mouse fibroblasts, human breast and choriocarcinoma, rat pancreas, rat pituitary tumor, rat villus crypt, human hepatocytes, rat hepatoma, monkey kidney, baby hamster ovary, and rat lung, due to the many variables in biologic materials, preparation and operating parameters, specific performance with other types of cells cannot be forecast.

The following examples illustrate the present invention without, however, limiting the same thereto.

## EXAMPLE 1 (comparative)

A unit of capillary bundles (Vitafiber® Amicon hollow-capillary unit 3P10) having a 10,000 molecular weight cut-off point, a 1,000 ml reservoir bottle, a peristaltic pump and Silastic tubing (about 2 meters) are autoclaved and assembled under aseptic conditions in the following manner:

The extracapillary space (2 ml) is charged with a suspension of $7.5 \times 10^6$ cells of a freshly harvested human hepatoma cell line (PLC/PRF/5, MacNab et al., Br. J. Cancer (1976) *34*, 509—515, a culture of which has been deposited with American Type Culture Collection and given accession number CCL 8024. The unit is placed in a 37°C $CO_2$ incubator. Every 30 minutes the hollow fiber unit is turned 180° around its longest axis. After 2 hours Eagle's Minimum Essential Medium (EMEM) containing 10% fetal calf serum, L-glutamine 2 mM, and Neomycin 50 $\mu$g/ml is circulated through the capillary unit at a flow rate of 5 ml/minute. After 2 weeks at 37°C the temperature of the incubator is reduced and maintained at 32°C and $10^{-4}$M caffeine is added. Cell growth is monitored by glucose utilization. Antigen samples are taken from the extracapillary space and assayed by complement fixation. The results are summarized in the following table:

| Age of cell culture (days) | Complement fixation titer |
|---|---|
| 7 | 4 |
| 14 | 16 |
| 21 | 64 |
| 28 | 512 |
| 35 | 512 |

Elimination of the fetal calf serum does not have any significant effect on titers and facilitates further purification of the HBsAg. Conventional monolayer tissue culture systems under similar conditions produced only traces of antigen.

## EXAMPLE 2

Three units of capillary bundles (Vitafiber®, Amicon) one 3P10, one P30 and one 3S100 having respectively molecular weight cut-off points of 10,000; 30,000 and 100,000 are each charged with a suspension of $6.0 \times 10^6$ cells of a freshly harvested higher HBsAg yielding clone of PLC/PRF/5 cells. The units, set-up as described in Example 1, are placed in a 37°C incubator. After 2 hours Eagle's Minimum Essential Medium (EMEM) containing 10% fetal calf serum, L-glutamine 2mM, and Neomcyin 50 $\mu$g/ml is circulated through the capillary unit at a flow rate of 5 ml/minute. After 2 weeks at 37°C the temperature of the incubator is reduced and maintained at 32°C and $10^4$M caffeine is added. Cell growth is monitored by glucose utilization. Antigen samples are taken from the extracapillary space and assayed by complement fixation. Samples from the circulating fluid are assayed by an enzyme immunoassay (AUS2YME™, Abbott Labs). The results are summarized in the following table:

| Day | 10,000 MW | 30,000 MW | 100,000 MW |
|---|---|---|---|
| 14 | 16 | 4 | 4 |
| 21 | 64 | 64 | 8 |
| 28 | 28 | 64 | 32 |
| 33 | 256 | 64 | 16 |
| 39 | 256 | 128 | 32 |

The unit with 10,000 molecular weight cut-off point proves superior yields although glucose consumption is similar in all three units.

4

## Claims

1. A method for preparing hepatitis B surface antigen which comprises growing cells which shed hepatitis B surface antigen in the presence of a nutrient medium on a permeable membrane having a molecular weight cut-off of about 10,000.

2. A method according to claim 1 wherein the growing is effected with two sequential stages having differing temperatures, each temperature being above room temperature with the first temperature being above the second temperature.

3. A method according to claim 2 wherein the first temperature is from 35 to 38°C.

4. A method according to claim 2 wherein the second temperature is from 30°C to 34°C.

5. A method according to claim 2 wherein the first temperature is about 37°C and the second temperature is about 32°C.

6. A method according to one of claims 1 to 5 wherein permeable membrane is a hollow fiber capillary unit.

7. A method according to one of claims 2 to 6 wherein caffeine is present during the second stage.

8. A method according to claim 7 wherein the caffeine is present in an amount from 0.0001 M to 0.0003 M.

## Revendications

1. Procédé pour préparer l'antigène du surface de l'hépatite B dans lequel on cultive des cellules qui excrètent l'antigène de surface de l'hépatite B en présence d'un milieu nutritif sur une membrane perméable ayant un seuil de coupure de poids moléculaire d'environ 10 000.

2. Procédé selon la revendication 1 où la culture s'effectue en deux stades successifs avec des températures différentes, chacune des températures étant supérieure à la température ambiante, et la première étant supérieure à la seconde.

3. Procédé selon la revendication 2 où la première température est comprise entre 35 et 38°C.

4. Procédé selon la revendication 2 où la seconde température est comprise entre 30° et 34°C.

5. Procédé selon la revendication 2 où la première température est d'environ 37°C et la seconde température est d'environ 32°C.

6. Procédé selon l'une des revendications 1 à 5 où la membrane perméable est une unité capillaire de fibre creuse.

7. Procédé selon l'une des revendications 2 à 6 où de la caféine est présente au cours de la seconde étape.

8. Procédé selon la revendication 7 où la caféine est présente en une quantité allant de 0,0001 M à 0,0003 M.

## Patentansprüche

1. Verfahren zur Herstellung des Hepatitis B Oberflächenantigens durch Wachstum von Zellen, die Hepatitis B Oberflächenantigen abgeben, in Anwesenheit eines Nährmediums auf einer permeablen Membran mit einer Molekulargewichts-Abtrennung von etwa 10 000.

2. Verfahren nach Anspruch 1, bei dem das Wachstum mit zwei aufeinander folgenden Stufen mit verschiedenen Temperaturen bedingt wird, wobei jede Temperatur über der Raumtemperatur liegt, und die erste Temperatur über der zweiten Temperatur liegt.

3. Verfahren nach Anspruch 2, bei dem die erste Temperatur 35 bis 38°C beträgt.

4. Verfahren nach Anspruch 2, bei dem die zweite Temperatur 30 bis 34°C beträgt.

5. Verfahren nach Anspruch 2, bei dem die erste Temperatur etwa 37°C und die zweite Temperatur etwa 32°C beträgt.

6. Verfahren nach einem der Ansprüche 1 bis 5, bei dem die permeable Membran einer Hohlfaser-Kapillareinheit ist.

7. Verfahren nach einem der Ansprüche 2 bis 6, bei dem während der zweiten Stufe Coffein anwesend ist.

8. Verfahren nach Anspruch 7, bei dem das Coffein in einer Menge von 0,0001 m bis 0,0003 m vorhanden ist.